# EUROPÄISCHE PATENTSCHRIFT

(11) **EP 0 575 732 B1**
(45) Veröffentlichungstag und Bekanntmachung des Hinweises auf die Patenterteilung: **18.12.1996**
(21) Anmeldenummer: 93107184.9
(22) Anmeldetag: 04.05.1993
(51) Int. Cl.: A61B 1/12, A61B 1/00, A61B 1/06

(54) **Abwinkelbarer Applikator für Lichtstrahlung für medizinische Zwecke**
Flexible illuminating device for medical applications
Dispositif d'illumination à courbure commandée destiné à des applications médicales

(30) Priorität: 17.06.1992 DE 4219807
(43) Veröffentlichungstag der Anmeldung: 29.12.1993
(73) Patentinhaber: AESCULAP AG, D-78501 Tuttlingen (DE)
(72) Erfinder: Wieneke, Paul, W-7200 Tuttlingen (DE)
(74) Vertreter: Hoeger, Stellrecht & Partner

(56) Entgegenhaltungen:
- DE-A- 3 707 899
- DE-U- 9 202 246
- DE-U- 9 208 125
- FR-A- 2 278 305
- US-A- 5 018 506

## Beschreibung

Die Erfindung betrifft einen abwinkelbaren Applikator für Lichtstrahlung für medizinische Zwecke, bestehend aus einer Lichtleitfaser zur Führung der Lichtstrahlung und einem die Lichtleitfaser aufnehmenden, schlauchförmigen aus einem in Längsrichtung dehnbaren Material bestehenden Mantel, siehe z.B. DE-U-9 202 246.

Für Behandlungen oder Untersuchungen mit Hilfe von Lichtstrahlung in sehr engen Körperhöhlen werden Lichtleitfasern verwendet, die die Lichtstrahlung zum entsprechenden Behandlungs- oder Untersuchungsgebiet führen und zu diesem Zweck in die entsprechenden Körperhöhlen vorgeschoben werden müssen. Als Lichtstrahlung wird beispielsweise Kaltlicht zur Beleuchtung von Körperinnenräumen oder Infrarotlicht zur Bestrahlung von krankhaft wucherndem Gewebe in Körperhöhlen verwendet. Dazu werden üblicherweise entsprechende Katheter in eine Körperöffnung eingeführt, und diese Katheter bilden einen Arbeitskanal, in den die Lichtleitfasern reversibel eingeschoben und aus dem sie nach Gebrauch wieder entfernt werden (US-A-4 807 593).

Um das gezielte Erreichen eines bestimmten Behandlungs- oder Untersuchungsgebietes, beispielsweise das Abbiegen in einer bestimmten Richtung am Ende eines engen Kanals, zu gewährleisten, werden für bekannte Katheter abwinkelnde Schuhe verwendet oder sie bestehen aus Röhren aus Spiraldraht, die mit einem Albaranhebel abgelenkt werden können. Alle diese starre mechanische Elemente verwendenden Konstruktionen sind allerdings nur dann einsetzbar, wenn die äußeren Dimensionen dieser Katheter einen bestimmten Wert nicht unterschreiten. Gerade bei Einsatz in kleinsten Räumen und Spalten ist jedoch ein sehr kleiner Systemdurchmesser für Applikatoren von Lichtstrahlung von sehr großer Bedeutung, um den anatomischen Größen gerecht zu werden.

Ein weiterer Nachteil der starre mechanische Elemente verwendenden Applikatoren für Lichtstrahlung ist die häufig ungleichmäßige Abwinklung der im Arbeitskanal verlaufenden Lichtleitfasern, die zum Bruch der Lichtleitfaser oder zu ungewollter Auskopplung der Lichtenergie aus der Lichtleitfaser durch die zu kleinen Biegeradien führen kann. Nur mit Abwinkelvorrichtungen, die einen relativ konstanten Biegeradius erzeugen, lassen sich Bruch oder ungewollte Auskopplung der Lichtenergie vermeiden.

Es sind auch biegsame Katheter bekannt, in die ein Lichtfaserbündel eingebettet ist. Diese Katheter sind dadurch biegbar ausgebildet, daß längenveränderliche Elemente im Katheter angeordnet sind, die durch elektrischen Strom betätigbar sind (US-A-4 934 340). Diese bekannten Katheter haben einen außerordentlich komplizierten Aufbau, da sie einerseits zwei elektrische Leiter aufnehmen müssen, von denen mindestens einer unter dem Einfluß des elektrischen Stromes längenveränderlich ist, und da außerdem Lichtfaserbündel eingebettet sein müssen, um die Strahlung an den gewünschten Ort zu transportieren. Dadurch ist es nicht möglich, die Dimensionen soweit zu reduzieren, wie es notwendig wäre, um den Katheter auch in engste Körperhöhlen zuverlässig einführen zu können.

Es ist Aufgabe der Erfindung, einen abwinkelbaren Applikator für Lichtstrahlung für medizinische Zwecke so auszubilden, daß er einen sehr kleinen Systemdurchmesser aufweist und einen konstanten Biegeradius erzeugt, so daß der Bruch der Lichtleitfaser oder die ungewollte Auskopplung der Lichtenergie aus der Lichtleitfaser durch zu kleine Biegeradien vermieden werden.

Diese Aufgabe wird bei einem Applikator für Lichtstrahlung der eingangs beschriebenen Art erfindungsgemäß dadurch gelöst, daß die Lichtleitfaser mit dem Mantel exzentrisch und eine Längsdehnung des Mantels im Kontaktbereich zwischen Lichtleitfaser und Mantel verhindernd verbunden ist und daß der Mantel eine am distalen Ende des Applikators verschlossene Kammer aufweist, die mit einer durch den Applikator verlaufenden Zufuhrleitung für ein fließfähiges Füllmedium verbunden ist.

Bei diesem Applikator für Lichtstrahlung dient also die Lichtleitfaser sowohl zur Führung der Lichtstrahlung an den Einsatzort als auch zur exzentrischen Längenfixierung des ansonsten in Längsrichtung dehnbaren Mantels. Wird die am distalen Ende des Applikators verschlossene Kammer durch Zuführung eines flüssigen oder gasförmigen Füllmediums mit Druck beaufschlagt, so bewirkt diese Druckerhöhung aufgrund der seitlichen Längenfixierung des Mantels durch die in ihrer Länge unveränderliche Lichtleitfaser eine einseitige Längsdehnung des Mantels und damit ein Abbiegen desselben.

Damit erhält man einen abwinkelbaren Applikator für Lichtstrahlung, der einen konstanten Biegeradius erzeugt und einen sehr kleinen Systemdurchmesser aufweist. Da der abwinkelbare Applikator zusätzlich von außen um seine Längsachse drehbar ist, erreicht man eine volle Beweglichkeit in einem sehr großen Raumwinkelbereich. Der Biegeradius ist durch Wahl des Druckes, den das Füllmedium auf den Mantel ausübt, in einem weiten Bereich variierbar. Bei Druckbeaufschlagung der Kammer durch das Füllmedium ist die Abbiegung des Applikators um so stärker ausgeprägt, je größer der diametrale Abstand zwischen der im Mantel angeordneten Kammer und der Lichtleitfaser ist.

Zwar sind bereits flexible Katheter bekannt, die hydraulisch in verschiedene Richtungen abgebogen werden können. Sie sind aus einem in Längsrichtung dehnfähigen Material hergestellt und weisen seitlich versetzte Kammern auf, die bei ungleichmäßiger Füllung der einzelnen Kammern mit einem Medium die Katheter so einseitig in Längsrichtung dehnen, daß sie eine Abbiegung erfahren (US-A-3 773 034). Diese Katheter bilden jedoch nur einen Arbeitskanal, durch die nach dem Einfügen der Katheter in eine Körperöffnung in einem weiteren Arbeitsschritt medizinische Instrumente, wie zum Beispiel Scheren oder pinzetten, an einen gewünschten Einsatzort durchgeschoben werden können. Der Arbeitskanal verläuft dabei jeweils zentral entlang der Längsachse der Katheter. Bedingt durch ihren komplizierten Aufbau weisen diese Katheter einen beträchtlichen Systemquerschnitt auf.

Günstig ist es, wenn der Mantel die Form eines Hohlzylinders aufweist und die Lichtleitfaser in den Bereich der Wand des Mantels eingefügt ist. Dies bewirkt eine besonders exzentrische Anordnung der Lichtleitfaser und einen besonders großen, eine Längsdehnung des Mantels verhindernden Kontaktbereich zwischen Lichtleitfaser und Mantel.

In diesem Fall ist es besonders vorteilhaft, wenn die Lichtleitfaser vollständig von einer Schicht des Mantelmaterials umgeben ist und die Kammer einen nierenförmigen Querschnitt aufweist. Dadurch wird ein bezüglich der Längsachse des Applikators besonders asymmetrischer Aufbau der Kammer bewirkt; dies wiederum hat ebenso wie die durch die Schicht erzeugte einseitige Verdickung des Mantels eine besonders hohe Empfindlichkeit des Biegeradius auf den Füllungsdruck des Füllmaterials zur Folge. Zusätzlich wird die Lichtleitfaser durch die sie vollständig umgebende Schicht des Mantelmaterials rundum vor äußeren Einflüssen geschützt.

In einer besonderen Ausführungsform des Applikators kann vorgesehen sein, daß sich die Kammer über die gesamte Länge des Applikators erstreckt, wodurch eine besonders einfache und kostengünstige Herstellung ermöglicht wird.

In einer weiteren Ausführungsform kann vorgesehen sein, daß der Mantel nur in einem dem distalen Ende benachbarten Bereich in Längsrichtung dehnbar ausgebildet ist. Dadurch wird erreicht, daß sich der Applikator nur in einem dem distalen Ende benachbarten Bereich abwinkelt, ansonsten aber eine gerade Form beibehält, was für bestimmte Anwendungen zweckdienlich sein kann.

Es kann auch vorgesehen sein, daß der Mantel über seine gesamte Länge dehnbar ausgestaltet ist und in einem dem proximalen Ende benachbarten Bereich von einer festen, eine Längsdehnung des Mantels verhindernden Hülle eng umschlossen ist. Dadurch wird mit einem besonders einfachen und somit kostengünstigen Aufbau erreicht, daß der Mantel nur in einem bestimmten, dem distalen Ende benachbarten Bereich dehnbar und damit abwinkelbar ist.

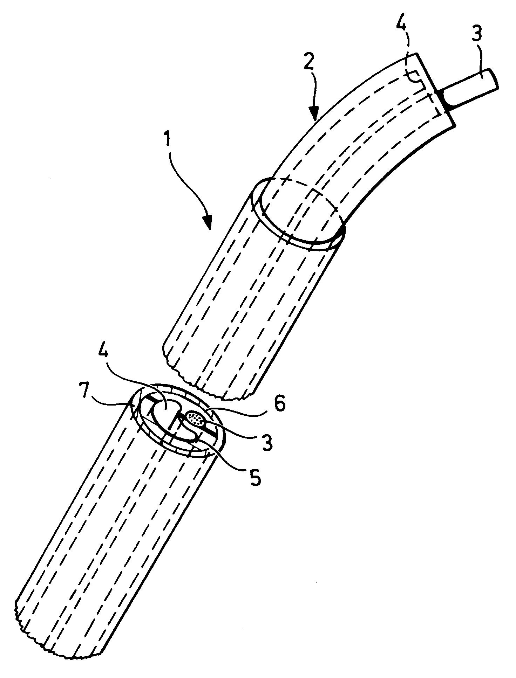

Die folgende Beschreibung einer bevorzugten Ausführungsform der Erfindung dient im Zusammenhang mit der Zeichnung der näheren Erläuterung. Die Zeichnung zeigt das freie Ende eines im Bereich des distalen Endes abgewinkelten Applikators für Lichtstrahlung.

Der in der Zeichnung dargestellte Applikator 1 umfaßt einen schlauchförmigen Mantel 2 mit einer Innenfläche 5 und einer Außenfläche 6, der aus einem in Längsrichtung dehnbaren Material besteht, beispielsweise aus einem Silikonmaterial. Über die gesamte Länge des Applikators ist in den im wesentlichen ringförmigen Bereich zwischen der Innenfläche 5 und der Außenfläche 6 des Mantels 2 eine Lichtleitfaser 3 eingebettet, deren distales Ende über das ansonsten vollkommen verschlossene distale Ende des Mantels 2 herausragt. In einem anderen, in der Zeichnung nicht gezeigten Ausführungsbeispiel, kann die Lichtleitfaser 3 zur Vermeidung von Verletzungen auch direkt mit dem Mantel abschließen. Die Innenfläche 5 des Mantels 2 und dessen verschlossenes distales Ende begrenzen eine sich über die gesamte Länge des Applikators erstreckende Kammer 4, die über eine in der Zeichnung nicht dargestellte Zufuhrleitung mit einem flüssigen oder gasförmigen Füllmedium beaufschlagt werden kann. Die Lichtleitfaser 3 wird im gesamten Bereich des Mantels 2 von einer Schicht des Mantelmaterials umgeben; dies hat zur Folge, daß die Kammer 4 einen nierenförmigen Querschnitt aufweist.

Bis auf einen dem distalen Ende benachbarten Bereich wird der Mantel 2 vom proximalen Ende des Applikators ausgehend von einer in ihrer Länge unveränderlichen Hülle 7 eng umschlossen, so daß der Mantel 2 nur in dem dem distalen Ende benachbarten Bereich in seiner Längsrichtung dehnbar ist.

Die in ihrer Länge unveränderliche Lichtleitfaser 3 führt zum einen die Lichtstrahlung an den gewünschten Einsatzort innerhalb einer eventuell sehr engen Körperhöhle, zum anderen bewirkt sie, daß sich der Mantel im unmittelbaren Kontaktbereich zwischen der Lichtleitfaser und dem Mantelmaterial in seiner Länge nicht verändern kann. Wird durch Zufuhr eines gasförmigen oder flüssigen Füllmediums in die Kammer 4 ein Druck auf den Mantel 2 ausgeübt, so führt die Fixierung des Mantels an die exzentrisch angeordnete Lichtleitfaser zu einer einseitigen Längenausdehnung des Mantels und damit zu einem Abbiegen des Applikators zu der Seite, an der die Lichtleitfaser angeordnet ist. Da der Mantel 2 zusätzlich von seinem proximalen Ende ausgehend an die in ihrer Länge unveränderliche Hülle 7 fixiert ist, kann sich der Mantel nur in dem dem distalen Ende benachbarten Bereich abbiegen. Durch Wahl der Länge der Hülle 7 wird somit ein definierter Abbiegebereich des Mantels erreicht.

## Patentansprüche

1. Abwinkelbarer Applikator (1) für Lichtstrahlung für medizinische Zwecke, bestehend aus einer Lichtleitfaser (3) zur Führung der Lichtstrahlung und einem die Lichtleitfaser (3) aufnehmenden, schlauchförmigen aus einem in Längsrichtung dehnbaren Material bestehenden Mantel (2),
dadurch gekennzeichnet, daß
die Lichtleitfaser (3) mit dem Mantel (2) exzentrisch und eine Längsdehnung des Mantels (2) im Kontaktbereich zwischen Lichtleitfaser (3) und Mantel (2) verhindernd verbunden ist und daß der Mantel (2) eine am distalen Ende des Applikators verschlossene Kammer (4) aufweist, die mit einer durch den Applikator (1) verlaufenden Zufuhrleitung für ein fließfähiges Füllmedium verbunden ist.

2. Applikator nach Anspruch 1, dadurch gekennzeichnet, daß die Lichtleitfaser (3) im Bereich zwischen der Innenfläche (5) und der Außenfläche (6) des Mantels geführt wird.

3. Applikator nach Anspruch 2, dadurch gekennzeichnet, daß die Lichtleitfaser (3) vollständig von einer Schicht des Mantelmaterials umgeben ist und die Kammer (4) einen nierenförmigen Querschnitt aufweist.

4. Applikator nach einem der voranstehenden Ansprüche, dadurch gekennzeichnet, daß sich die Kammer (4) über seine gesamte Länge erstreckt.

5. Applikator nach Anspruch 4, dadurch gekennzeichnet, daß der Mantel (2) nur in einem dem distalen Ende benachbarten Bereich in Längsrichtung dehnbar ausgebildet ist.

6. Applikator nach Anspruch 4, dadurch gekennzeichnet, daß der Mantel (2) über seine gesamte Länge dehnbar ausgestaltet ist und in einem dem proximalen Ende benachbarten Bereich von einer festen, eine Längsdehnung des Mantels verhindernden Hülle (7) eng umschlossen ist.

## Claims

1. A flexible illuminating applicator (1) for medical use, comprising an optical fibre (3) for guiding the beam of light and a tubular sheath (2) comprising a material extensible in the longitudinal direction and receiving the optical fibre (3), characterised in that the optical fibre (3) is eccentrically connected to the sheath (2) so as to prevent longitudinal extension of the sheath (2) in the contact region between the optical fibre (3) and the sheath (2), and in that the sheath (2) has a chamber (4) closed at the distal end of the applicator and connected to a feed line extending through the applicator (1) and provided for a fluid filling medium.

2. An applicator according to claim 1, characterised in that the optical fibre (3) is guided in the region between the inner surface (5) and the outer surface (6) of the sheath.

3. An applicator according to claim 2, characterised in that the optical fibre (3) is completely surrounded by a layer of the sheath material and the chamber (4) has a kidney-shaped cross-section.

4. An applicator according to any one of the preceding claims, characterised in that the chamber (4) extends over the entire length of the applicator.

5. An applicator according to claim 4, characterised in that the sheath (2) is formed so as to be extensible in the longitudinal direction only in a region adjacent to the distal end.

6. An applicator according to claim 4, characterised in that the sheath (2) is formed so as to be extensible over its entire length and, in a region adjacent to the proximal end, is closely surrounded by a rigid casing (7) preventing longitudinal extension of the sheath.

## Revendications

1. Applicateur (1) de rayonnement lumineux coudable pour usage médical, composé d'une fibre optique (3) destinée à conduire le rayonnement lumineux et d'une gaine (2) tubulaire en un matériau extensible dans le sens longitudinal, logeant la fibre optique (3), caractérisé en ce que la fibre optique (3) est liée à la gaine (2) de façon excentrique et empêchant un allongement longitudinal de la gaine (2) dans la zone de contact entre la fibre optique (3) et la gaine (2) et en ce que la gaine (2) présente une chambre fermée (4) à l'extrémité distale de l'applicateur, laquelle communique avec un conduit d'amenée de milieu de remplissage fluide traversant l'applicateur (1).

2. Applicateur selon la revendication 1, caractérisé en ce que la fibre optique (3) est disposée dans la zone entre la surface interne (5) et la surface externe (6) de la gaine.

3. Applicateur selon la revendication 2, caractérisé en ce que la fibre optique (3) est totalement entourée par une couche du matériau de la gaine et que la chambre (4) présente une section transversale réniforme.

4. Applicateur selon l'une des revendications précédentes, caractérisé en ce que la chambre (4) s'étend sur toute sa longueur.

5. Applicateur selon la revendication 4, caractérisé en ce que la gaine (2) n'est extensible dans le sens longitudinal que dans une zone proche de l'extrémité distale.

6. Applicateur selon la revendication 4, caractérisé en ce que la gaine (2) est extensible sur toute sa longueur et est étroitement entourée, dans une zone proche de l'extrémité proximale, par une enveloppe (7) solide, empêchant un allongement longitudinal de la gaine.
